# EUROPEAN PATENT APPLICATION

(11) **EP 1 466 895 A1**
(43) Date of publication of application: **13.10.2004**
(21) Application number: 03701750.6
(22) Date of filing: 17.01.2003
(51) Int. Cl.: C07C 309/66, C07C 303/30, C07C 327/32

(54) **PROCESS FOR PRODUCING 2-ARALKYLPROPIONIC ACID DERIVATIVE**

(30) Priority: 17.01.2002 JP 2002009259
(71) Applicant: KANEKA CORPORATION, Osaka-shi, Osaka 530-8288 (JP)
(72) Inventor: FUSE, Yoshihide, c/o KANEKA CORP. Takasago Plant, Takasago-shi, Hyogo 676--8688 (JP); KINOSHITA, Koichi, c/o KANEKA CORP. Takasago Plant, Takasago-shi, Hyogo 676-8688 (JP); KAWASAKI, Hiroaki, c/o KANEKA CORP. Takasago Plant, Takasago-shi, Hyogo 676-8688 (JP)
(74) Representative: HOFFMANN - EITLE
(86) International application number: PCT/JP2003/000329
(87) International publication number: WO 2003/059869

(57) **Abstract**

A process for easily and industrially advantageously producing a high-purity 2-aralkyl-3-acetylthiopropionic acid and a high-purity 2-aralkylpropionic acid having a leaving group in the 3-position from easily available compounds. A 2-aralkyl-1-propanol having a sulfonyloxy group or halogen atom in the 3-position is oxidized with a permanganate under acidic conditions to produce a high-purity 2-aralkylporpionic acid having sulfonyloxy or the halogen in the 3-position. This acid is reacted with a thioacetate in the presence of water to produce a high-purity 2-aralkyl-3-acetylthiopropionic acid.

## Description

### FIELD OF THE INVENTION

The present invention relates to a method for producing a 2-aralkylpropionic acid represented by Formula (2): 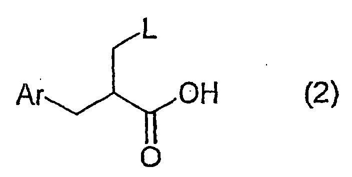 wherein Ar is an optionally substituted aryl group having 6 to 18 carbon atoms, and L is a sulfonyloxy group or a halogen atom, especially an optically active 2-aralkylpropionic acid and a 2-aralkyl-3-acetylthiopropionic acid represented by Formula (3): 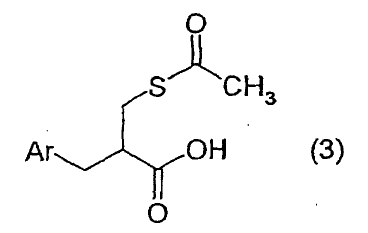 wherein Ar is an optionally substituted aryl group having 6 to 18 carbon atoms, especially an optically active 2-aralkyl-3-acetylthiopropionic acid. The compound is an extremely useful compound as an intermediate capable of being employed in the fields of pharmaceuticals and others, including an analgesic and a hypotensive agent having effects such as encephalinase inhibition and ACE inhibition.

### BACKGROUND OF THE INVENTION

A conventional method for producing an optically active 2-aralkyl-3-acetylthiopropionic acid may for example be a method by an addition reaction of thioacetic acid to a 2-aralkylacrylic acid (JP-A-2-157260, JP-A-62-270555, J. Med. Chem.37, 2461-2476(1994), which generally gives a racemic 2-aralkyl-3-acetylthiopropionic acid, due to which a procedure for isolating a desired optically active form using an optical resolution technology for the purpose of obtaining a pharmaceutically useful optically active form.

While various methods for the optical resolution of 2-aralkyl-3-acetylthiopropionic acids were reported (JP-A-11-228532, JP-A-8-59606, J. Med. Chem. 35, 602-608 (1992)), any of them exhibits poor optical resolution efficiency, employs an expensive optical resolution reagent, and requires a complicated procedure, thus having poor industrial usefulness.

As another method for producing an optically active 2-aralkyl-3-acetylthiopropionic acid, a method is disclosed in which a precursor of the compound having a leaving group in its 3-position, namely, an optically active 2-aralkylpropionic acid, is subjected to a replacement of the 3-position leaving group with thioacetic acid while keeping its steric structure. For example, the following methods:
(i) a method employing an acethylthio-derivatization in tetrahydrofuran utilizing a Mitsunobu reaction (azodicarboxylic acid diester/triphenylphosphine and thioacetic acid) starting from an optically active 2-benzyl-3-hydroxypropionic acid (JP-W-11-503470),
(ii) a method employing a reaction of an optically active 2-benzyl-3-chloropropionic acid with potassium thioacetate in the presence of potassium iodide in dimethylacetoamide to effect an acetylthio-derivatization (Aust. J. Chem., 51, 511-514, (1998)),
(iii) a method which brings an optically active 2-benzyl-3-methanesulfonyloxypropionic acid into contact with thioacetic acid and a base, or a potassium salt of thioacetic acid in methanol to effect an acetylthio-derivatization(WO98/05634), can be exemplified.

However, Method (i) requires a reagent which is expensive and can be handled only in a limited manner industrially, and its yield is 82% which is not necessarily satisfactory. In Method (ii), the yield is as extremely low as 54%, which poses a problem in its industrial use.

On the other hand, Method (iii) exhibits a higher reaction yield (about 95%) when compared with the methods described above, and can industrially be employed. Nevertheless, it was revealed, based on our detailed investigation by the present inventors, to allow various impurities such as a deacetylated form of the target compound, namely 2-aralkyl-3-mercaptopropionic acid (hereinafter referred to as a deacetylated form) and other impurities analogous thereto to be formed as by-products. Such a by-product may lead to a reduction in the quality and the yield of the target compound, thus posing a problem in the field of pharmaceutical production where the quality is expected to be raised by reducing even a small amount of the impurity.

Thus, it is still demanded to develop a convenient and industrially excellent method for producing an optically active 2-aralkyl-3-acetylthiopropionic acid in which the steric formation of the optically active 2-aralkylpropionic acid having a leaving group in its 3-position is maintained while the leaving group in the 3-position is substituted by thioacetic acid whereby achieving an efficient prevention of the by-product formation as well as high quality and yield.

On the other hand, a method for producing a 2-aralkylpropionic acid having a leaving group in its 3-position useful as an intermediated for producing the 2-aralkyl-3-acetylthiopropionic acid described above may for example be a method employing an oxidation of a 2-aralkyl-1-propanol having a leaving group in the 3-position. Those which can be exemplified are follows:
(iv) a method employing an oxidation of an optically active 2-benzyl-3-methanesulfonyloxy-1-propanol with an aqueous solution of sodium hypochlorite using 2,2,6,6-tetramethylpiperidine-1-oxy (TEMPO) derivative as a catalyst, and,
(v) a method using an oxidation with a Jones reagent (both shown above in WO98/05634),
(vi) a method using an oxidation of 2-benzyl-3-iodo-1-propanol with a Jones reagent (J. Am. Chem. Soc., 116, 7475-7480 (1994)).

However, Method (iv) can not be free of by products (about 1 to 3%) including a compound resulting from a halogenation of an aromatic ring by a halogen-based oxidizer employed and poses a requirement of an enormous effort for its removal to achieve a purification. Method (v) or (vi) employs a metal chromium compound. A metal chromium compound is not preferable in an industrial production because of the limitation in handling, an enormous load posed in treating or controlling the highly toxic waste, and possible hazardous effects on human and environment, and is disadvantageous also economically. Accordingly, the use of such a reagent in an industrial production should be avoided.

On the other hand, no oxidation processes other than those mentioned above has not been applied to a 2-aralkyl-1-propanol having a leaving group in the 3-position.

Under the circumstance described above, it has been desired to develop a convenient and industrially excellent method for producing a 2-aralkylpropionic acid having a leaving group in the 3-position, especially an optically active 2-aralkylpropionic acid having a leaving group in the 3-position while suppressing the by-product formation.

### SUMMARY OF THE INVENTION

Under the circumstance described above, an objective of the invention is to provide a method for producing a 2-aralkylpropionic acid having a leaving group in the 3-position and a 2-aralkyl-3-acetylthiopropionic acid, especially an optically active 2-aralkylpropionic acid having a leaving group in the 3-position and an optically active 2-aralkyl-3-acetylthiopropionic acid which are extremely useful production intermediates employed in pharmaceutical or other fields, at a high purity in a commercially and industrially advantageous manner.

The present inventors made an effort to solve the problems mentioned above and finally discovered that a highly pure 2-aralkylpropionic acid substituted by a leaving group in its 3-position can be produced at a high yield without forming an impurity as a by-product generated as a result of the halogenation of the aromatic ring described above and also without affecting the leaving group in the compound by oxidizing a 2-aralkyl-1-propanol having a leaving group in the 3-position using a permanganate under an acidic condition.

We also discovered that by reacting a 2-aralkylpropionic acid having a leaving group in the 3-position with thioacetate in the presence of water an extremely pure 2-aralkyl-3-acetylthiopropionic acid can be produced while limiting the formation of impurities as by-products to an extremely low level, thus establishing the present invention.

Thus the invention is a method for producing a 2-aralkylpropionic acid represented by Formula (2): 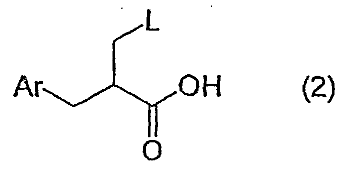 wherein Ar is an optionally substituted aryl group having 6 to 18 carbon atoms, and L is a sulfonyloxy group or a halogen atom, comprising oxidizing a 2-aralkyl-1-propanol represented by Formula (1): 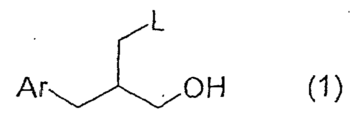 wherein Ar and L are as defined above, using a permanganate under an acidic condition.

Moreover, the invention is a method for producing a 2-aralkyl-3-acetylthiopropionic acid represented by Formula (3): 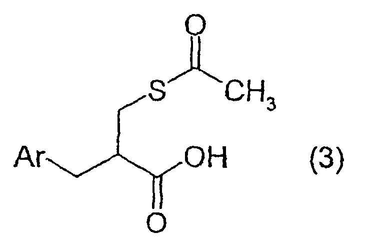 wherein Ar is as defined above, comprising reacting a 2-aralkylpropionic acid represented by Formula (2): 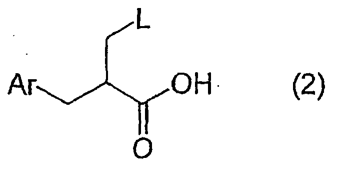 wherein Ar and L are as defined above, with a thioacetate in the presence of water.

### DETAILED DESCRIPTION OF THE INVENTION

The invention is detailed below.
The invention involves the following steps:
Step a) a 2-aralkyl-1-propanol represented by Formula (1): 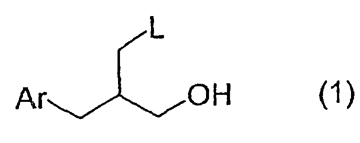 wherein Ar is an optionally substituted aryl group having 6 to 18 carbon atoms, and L is a sulfonyloxy group or a halogen atom is oxidized to produce a 2-aralkylpropionic acid represented by Formula (2): 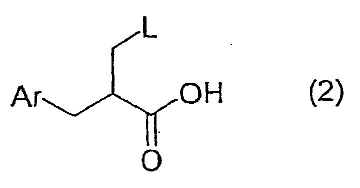 wherein Ar and L are as defined above; and,
Step b) the leaving group L in the compound represented by Formula (2) shown above is substituted by thioacetic acid to produce a 2-aralkyl-3-acetylthiopropionic acid represented by Formula (3): 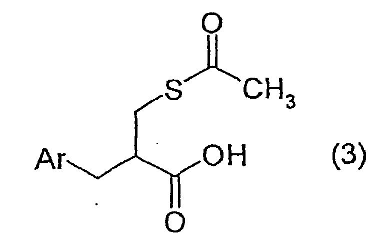 wherein Ar is as defined above.

In Compounds (1), (2) and (3) shown above, Ar denotes an optionally substituted aryl group having 6 to 18 carbon atoms.

While the aryl group mentioned above is not limited particularly, it may for example be an optionally substituted phenyl group or an optionally substituted naphthyl group. Such an aryl group is preferably a phenyl group optionally substituted by an alkyl group, substituted alkyl group, alkoxy group, substituted alkoxy group or a halogen atom, with phenyl group being more preferred.

In Compounds (1) and (2) shown above, a leaving group L denotes a sulfonyloxy group or a halogen atom.

While the sulfonyloxy group is not limited particularly, it is preferably an optionally substituted straight, branched or cyclic alkylsulfonyloxy group having 1 to 6 carbon atoms, or an optionally substituted arylsulfonyloxy group having 6 to 18 carbon atoms. A substituent on such a sulfonyloxy group may for example be a methyl group, halogen atom, nitro group and the like.

The alkylsufonyloxy group mentioned above may typically be a methanesulfonyloxy group, ethanesulfonyloxy group, trifluoromethanesulfonyloxy group and the like, while the arylsulfonyloxy group mentioned above may for example be a toluenesulfonyloxy group, benzenesulfonyloxy group, o-, p- or m-nitrobenzenesulfonyloxy group and the like. Among those listed above, a methanesulfonyloxy group or p-toluenesulfonyloxy group is preferred, with a methanesulfonyloxy group being more preferred.

When the leaving group L is a halogen atom, then the halogen atom may for example be a chlorine atom, bromine atom, iodine atom and the like, with a chlorine atom and bromine atom being preferred.

In Compounds (1) and (2) shown above, it is preferred that Ar is a phenyl group, and L is a methanesulfonyloxy group.

Each step is detailed below.

### Step a)

A 2-aralkyl-1-propanol (1) employed in this step having a leaving group such as a sulfonyloxy group or halogen atom in the 3-position can be obtained by a known method, for example a method described in WO98/05634, Synthesis, 1427-31 (1995), J. Am. Chem. Soc. 116, 7475-7480 (1994).

In this step, a 2-aralkyl-1-propanol represented by Formula (1) shown above having a leaving group in the 3-position is oxidized using a permanganate under an acidic condition to produce a 2-aralkylpropionic acid represented by Formula (2) shown above having a leaving group in the 3-position.

In this step, it is preferred to oxidize the compound (1) described above using a permanganate in an acidic aqueous solution. Typically, the compound (1) described above is added to the acidic aqueous solution, to which then the permanganate is added preferably in portions, whereby effecting an oxidizing reaction.

While the reaction is conducted generally in a solid-liquid heterogeneous system, it is also possible to use a permanganate as being solubilized for example by a crown polyether.

While a permanganate employed in the reaction described above is not limited particularly, an alkaline metal salt of permanganic acid is preferred, and those which can be exemplified are potassium permanganate and sodium permanganate, with potassium permanganate being preferred especially. Any of these may be used alone or in combination of two or more.

The amount of a permanganate in the reaction described above is generally 1 to 10 equivalents based on the reaction substrate compound (1), preferably 1.5 to 5 equivalents, more preferably 2 to 4 equivalent.

An acidic aqueous solution employed in the reaction described above is not limited particularly as long as it is an aqueous solution within a pH range defined ordinarily as acidic, usually being an aqueous solution at a pH lower than 7, preferably at pH6 or less, more preferably at pH5 or less.

Since an oxidizing reaction conducted using the permanganate described above generally allows the reaction solution to become alkaline gradually along with the advancement of the reaction, it is preferred, for the purpose of maintaining the reaction solution under an acidic condition, that an excessive acid is allowed to exist preliminarily or an acid is further added along with the advancement of the reaction to keep the acidic condition. A method for maintain the acidic condition by allowing a pH buffering substance such as phosphate, borate and acetate to coexist may also be employed preferably. Any of these methods can be used alone or in combination of two or more.

An acid employed in the acidic aqueous solution described above is not limited particularly, and may be an organic or inorganic acid by which the reaction is not affected adversely. The acid may be a weak acid or a strong acid.

Such an organic acid may typically be an aliphatic carboxylic acid such as acetic acid, propionic acid, butyric acid, trifluoroacetic acid and the like; an aromatic carboxylic acid such as benzoic acid and the like; a sulfonic acid such as methanesulfonic acid, trifluoromethanesulfonic acid and the like, with acetic acid being preferred. An inocrganic acid may typically be sulfuric acid, hydrochloric acid, phosphoric acid,boric acid, nitric acid and the like, with sulfuric acid being employed preferably. Any of these acids may be employed alone or in combination of two or more.

The amount of the acid mentioned above may be an amount capable of keeping the reaction mixture under an acidic condition. The amount is preferably 1 to 20-fold molar amount based on the permanganate described above, more preferably 1 to 10-fold molar amount, although it may vary depending on the type of the acid.

While some substrate in the reaction described above becomes in a suspension state when using an acidic aqueous solution as a solvent, a target compound can be obtained by conducting the reaction with adding a permanganate. It is also possible to use an organic solvent concomitantly to an extent which does not affect the reaction adversely, thus using a solvent mixture of an acidic aqueous solution and an organic solvent. The reaction is also conducted preferably that a substrate is dissolved in a solvent mixture to make solution state and a permanganate is added to this. While such an organic solvent is not limited particularly, it may be an organic solvent having a compatibility with water or an organic solvent having no compatibility with water. Any two or more of these organic solvents,regardless of the types, may be selected and used concomitantly.

As used herein, an organic solvent having a compatibility with water is an organic solvent which forms a homogeneous phase even at the time of cessation of the flowing after termination of mixing once after being agitated vigorously with an equal amount of water at 20°C. On the contrary, an organic solvent having no compatibility with water is one which forms a heterogeneous phase to yield two or more phases in the system under the same condition above.

First, a case using an organic solvent having a compatibility in combination with an acidic aqueous solution as a reaction solvent is detailed below.

An organic solvent employed here is not limited particularly, as long as it is an inert solvent, and may usually be tert-butanol, acetone, tetrahydrofuran, ethanol and the like. Among them, tert-butanol and acetone may be preferred. Any of these organic solvents may be employed alone or in combination of two or more.

The ratio between an acidic aqueous solution and an organic solvent having a compatibility with water, when represented as a weight ratio of the acidic aqueous solution/the organic solvent having a compatibility with water, ranges from 90/10 to 10/90, preferably, 80/20 to 20/80.

It is further preferred in the concomitant use mentioned above to use a solvent mixture system of an acidic aqueous solution consisting of sulfuric acid and water combined with acetone for the purpose of achieving a higher yield and a higher quality of a target material.

Next, a case using an organic solvent having no compatibility with water concomitantly as an acidic aqueous solution is detailed below. In such a case, said reaction becomes a biphasic reaction. Accordingly, the ratio between the acidic aqueous solution and the organic solvent having no compatibility with water may vary without limitation.

Preferred organic solvents having no compatibility with water are not limited particularly, and may for example be acetic esters such as ethyl acetate, propyl acetate, butyl acetate and the like; aliphatic hydrocarbons such as pentane, hexane, cyclohexane, heptane, isooctane, methylcyclohexane and the like; aromatic hydrocarbons such as benzene, toluene and the like; halogenated hydrocarbons such as dichloromethane, chloroform and the like; ketones such as methyl ethyl ketone, diisopropyl ketone and the like; ethers such as diethyl ether, diisopropyl ether, tert-butylmethyl ether and the like. An acetic alkyl ester having 1 to 6 carbon atoms is preferred, with ethyl acetate being preferred particularly. Any of these solvents may be employed alone or in combination of two or more.

In a reaction in a biphasic system described above, a phase transfer catalyst may be employed concomitantly. Such a phase transfer catalyst employed here is not limited particularly, and for example, a quaternary ammonium salt, which is a cationic activator, such as tetrabutylammonium chloride, tetrabutylammonium bromide, tricaprylylmethylammonium chloride, trioctylmethylammonium bromide and the like may be employed. Any of these substances may be employed alone or in combination of two or more.

The reaction temperature in the oxidizing reaction described above is not limited particularly as long as it allows the reaction to be advanced, and may be within the range from the boiling point to the freezing point of a solvent employed. The temperature is usually -30°C or higher and 40°C or below, preferably -20°C or higher and 30°C or below, although it may depend on the type of the reaction solvent. Relatively, this reaction is highly exothermic. Since the elevation of the reaction temperature leads to a reduced yield or reduced quality, the reaction is conducted preferably with cooling to 20°C or below, preferably 10°C or below for the purpose of controlling the reaction appropriately. On the other hand, the reaction is conducted preferably at a temperature of -10°C or higher from an industrial point of view since a lower reaction temperature poses a requirement of a longer time period for completion of the reaction.

Especially when using the biphasic reaction described above, a compound can readily be protected from the decomposition due to an elevated reaction temperature or a prolonged reaction time, and thus there is no need to pay any attention to the stabilization, giving a highly industrial advantage.

After completion of the oxidizing reaction described above, a target compound is separated from an excessive amount of manganese compounds such as permanganates and permanganate decomposition products which are present in the reaction mixture. While such a separation process may be conducted by a solid-liquid separation procedure such as a filtration, the present inventors established here a method for separating manganese compounds readily without needing any solid-liquid separation procedure by means of a treatment of the manganese compounds with a reducing agent. Thus, the treatment with the reducing agent under an acidic condition allows the manganese compounds in the reaction mixture described above to be dissolved in the aqueous phase, thus enabling the separation of the target compound from the manganese compounds even by a simple procedure such as the extraction/partition using an organic solvent.

When treating manganese compounds, a reducing agent employed is not limited particularly, and may for example be an aqueous sulfurous acid; and a sulfite such as sodium sulfite, potassium sulfite, ammonium sulfite and the like; a hydrogen sulfite such as sodium hydrogen sulfite, potassium hydrogen sulfite, ammonium hydrogen sulfite and the like; a pyrosulfite, such as sodium pyrosulfite, potassium pyrosulfite and the like; a dithionite such as sodium dithionite, ammonium dithionite and the like; a thiosulfate such as sodium thiosulfate, potassium thiosulfate, ammonium thiosulfate, calcium thiosulfate and the like; a nitrite such as sodium nitrite, potassium nitrite and calcium nitrite and the like; a carboxylic acid such as oxalic acid, glyoxylic acid and the like; as well as aqueous solutions thereof. Among those listed above, a hydrogen bisulfite, sulfite, pyrosulfite and aqueous solutions thereof are preferred. Any of these may be employed alone or in combination of two or more.

As used herein, an acidic condition is not limited particularly as long as it is at a pH defined ordinarily as acidic, and an acidic aqueous solution is also preferred, usually being an aqueous solution at a pH lower than 7. It is also preferred to use an optimum pH for the dissolution of a manganese compound. Such a pH is usually pH5 or lower, preferably pH4 or lower, more preferably pH3 or lower, although it may vary depending on the type of the reducing agent employed. Accordingly, upon adjusting the pH within the range specified above, a necessary amount of a mineral acid such as sulfuric acid or hydrochloric acid or an organic acid such as glyoxylic acid may be employed in the treatment. It may also be used an excessive amount.

Since a treatment with the reducing agent mentioned above is exothermic generally, it is preferable to use the reducing agent with controlling the treatment temperature. Usually, the treatment is preferably conducted at a temperature which is higher than freezing point of the solvent and not higher than 30°C.

For the purpose of a complete dissolution of a manganese compound, a reducing agent is used preferably in an amount usually of 1 to 3-fold molar amount based on the manganate employed, and once the exothermic reaction by the reducing agent ceased then the temperature is kept at 5°C or higher, not higher than the boiling point of the solvent employed, preferably at 10°C or higher but not higher than the boiling point of the solvent employed.

After a manganese compound is dissolved in an aqueous phase by the treatment mentioned above, an organic solvent phase (extract) containing a target compound is obtained by an extraction with the organic solvent. It is also preferred to reduce or eliminate any manganese compound by washing the organic solvent phase further with water.

An extract containing a target substance obtained by the method described above is concentrated to distill the solvent off to yield the target substance. While the target substance thus obtained is almost pure, it is preferred that it may further be purified by an ordinary procedure such as crystallization and column chromatography.

The followings are the description of Process b).

### Process b)

A 2-aralkylpropionic acid (2) having a sulfonyloxy group or a halogen atom in the 3-position employed in this step is preferably one produced by the method described in Step a). Otherwise, it may be one produced by a known method, such as a method described in WO98/05634, WO98/05635, JP-A-7-316094, Aust. J. Chem. 51, 511-514 (1998), Chemische. Berichte. 123, 635-638 (1990), J. Am. Chem. Soc. 116, 7475-7480 (1994) and the like.

In this step, the compound (2) described above is reacted with a thioacetate in the presence of water to produce the 2-aralkyl-3-acetylthiopropionic acid (3) described above.

This reaction is conducted in the presence of water. The reaction in the presence of water limits the by-product formation to a reduced level and gives a higher yield and a higher quality of a target compound (3). The formation of by-products, especially, a deacetylated form of the target compound(3), namely 2-aralkyl-3-mercaptopropionic acid (hereinafter also referred to as a deacetylated form) and analogous impurities and other impurities can effectively be suppressed, and the production of the target compound at a high purity and a high yield becomes possible.

A way for allowing water to exist is conveniently the use of water as a sole solvent, which maximizes the effect of water on suppressing the formation of by-product, and water may be used also in a solvent mixture containing an organic solvent in an amount by which the reaction is not affected adversely, or, alternatively, the reaction solvent may be used an organic solvent, to which water is added to serve as a solvent mixture.

An organic solvent employed here is not limited particularly, and may be an organic solvent having a compatibility with water or an organic solvent having no compatibility with water. The organic solvent may be a protic solvent or an aprotic solvent. As used herein, "an organic solvent having a compatibility with water" and "an organic solvent having no compatibility with water" are those defined respectively in Step a) described above.

When using a solvent mixture of water and an organic solvent having a compatibility with water, it is preferred to use a higher ratio of water. In such a case, a higher ratio of water gives a higher ability of suppressing a by-product formation, resulting in a target compound (3) having a higher purity.

The ratio between water and an organic solvent having a compatibility with water may depend on the type of the organic solvent and the quality of the target compound, and may usually be 10/90 or higher when represented as the weight ratio of water/the organic solvent having a compatibility with water, more preferably 20/80 or higher, especially 30/70 or higher. It is also preferable that the ratio of water/the organic solvent having a compatibility with water is set at 50/50 or higher to obtain a further purer target compound (3).

The type of an organic solvent having a compatibility with water described above is not limited particularly, and may be selected from the organic solvents by which said reaction is not affected adversely. Those exemplified typically are alcohols such as methanol, ethanol, propanol, isopropanol, ethylene glycol, methoxyethanol and the like; ethers such as tetrahydrofuran, 1,4-dioxane and the like; amides such as dimethylformamide, dimethylacetoamide, N-methyl-2-pyrrolidone and the like; nitriles such as acetonitrile and the like; sulfoxides such as dimethyl sulfoxide and the like; ketones such as acetones and the like; phosphoryl amide such as hexamethylphosphoryl triamide and the like. Among these organic solvents, a protic solvent is preferred for the purpose of obtaining a target compound having a higher quality, such as an alcohol, especially a lower alcohol having a small number of carbon atoms, particularly an alcohol having 1 to 3 carbon atoms, most preferably methanol. Any of these organic solvents may be employed alone or in combination of two or more.

Next, a case using a solvent mixture system consisting of water and an organic solvent having no compatibility with water is discussed. In such a case, the reaction becomes a biphasic reaction. Accordingly, a maximal by-product formation suppressing effect of water is obtained similarly to a case of the reaction in water as a single solvent, and the ratio between water and the organic solvent having no compatibility with water may vary without limitation. In addition, the concomitant use of the organic solvent having no compatibility with water was further revealed to give a further preferable property in view of the by-product suppression in the reaction. Thus, the concomitant use of the organic solvent having no compatibility with water allows a phase transfer reaction mode to be established, resulting in an effective inhibition of the by-product formation due for example to a decomposition reaction, which leads to an additive contribution to a further promoted by-product formation suppressing effect in this reaction.

A preferred organic solvent having no compatibility with water is not limited particularly, and includes aromatic hydrocarbons such as toluene, xylene, benzene and the like; acetic esters such as ethyl acetate, propyl acetate, butyl acetate and the like; hydrocarbons such as pentane, hexane, cyclohexane, heptane and the like; halogenated hydrocarbons such as dichloromethane, chloroform and the like; ethers such as diethyl ether, diisopropyl ether, dibutyl ether, tert-butyl methyl ether and the like. Among those listed above, aromatic hydrocarbons or acetic alkyl ester having 1 to 6 carbon atoms is preferred, with toluene and ethyl acetate being more preferred. Any of these may be employed alone or in combination of two or more.

In the biphasic reaction described above, a phase transfer catalyst may also be employed. Such a phase transfer catalyst employed here is not limited particularly, and the reaction can be conducted with adding a catalytic amount of a quaternary ammonium salt described in Step a).

The reaction temperature here is not limited particularly, and may be within the range from the boiling point to the freezing point of the solvent system employed. Typically, the temperature, from an industrial point of view, is usually -20°C or higher and 80°C or below, preferably -10°C or higher and 70°C or below, more preferably 0°C or higher and 60°C or below, although it may depend on the type of the reaction solvent employed. A lower temperature of this reaction leads to a longer time period required for completion of the reaction. From an industrial point of view and for the purpose of completing the reaction within 24 hours, the reaction is conducted preferably at 20°C or higher, and a favorable reaction can be conducted at about 40°C.

A thioacetate employed in this reaction is subjected to the reaction preferably in the form of its salt. From this point of view, it is preferable to use a thioacetate. Such a thioacetate is not limited particularly, and may for example be an alkaline metal salt of thioacetic acid such as sodium thioacetate, potassium thioacetate, lithium thioacetate, cesium thioacetate and the like; an alkaline earth metal salt of thioacetic acid such as calcium thioacetate, magnesium thioacetate, barium thioacetate and the like; an amine salt of thioacetic acid such as ammonium thioacetate and the like. Industrially, an alkaline metal salt of thioacetic acid is preferred, with sodium or potassium salt of thioacetic acid being more preferred. Any of these may be employed alone or in combination of two or more.

The amount of a thioacetate employed is not limited particularly, and usually 1 to 3 equivalents based on a substrate, preferably 1 to 2 equivalents, with 1.5 equivalents being most preferred for obtaining a higher quality.

It is also possible to form the thioacetate described above in the reaction system using thioacetic acid and a base. While in a general procedure when forming a thioacetate in the reaction system a base is added a solution of thioacetic acid to form a thioacetate and then a substrate is added and reacted, it is also possible to add a base to a solution containing thioacetic acid and a substrate to effect the reaction with forming a thioacetate. When a base is added to a solution containing thioacetic acid and a substrate, it is also preferred that the pH which naturally varies along with the advancement of the reaction is maintained within the optimum pH range for the reaction with adding a base continuously to effect the reaction. In such a case, a pH buffering agent such as phosphate, borate, acetate and the like may coexist.

A base employed here is not limited particularly and may for example be an alkoxyalkaline metal salt such as sodium methoxide, potassium methoxide, sodium ethoxide, potassium ethoxide, potassium t-butoxide and the like; an alkaline metal carbonate such as lithium carbonate, sodium carbonate, potassium carbonate, cesium carbonate and the like; an alkaline metal hydrogen carbonate such as lithium hydrogen carbonate, sodium hydrogen carbonate, potassium hydrogen carbonate, cesium hydrogen carbonate and the like; an alkaline earth metal carbonate such as calcium carbonate, barium carbonate and the like; an alkaline metal hydroxide such as lithium hydroxide, sodium hydroxide, potassium hydroxide, cesium hydroxide and the like; a hydroxylated alkaline earth metal salt such as calcium hydroxide, barium hydroxide and the like; a tertiary amine such as triethylamine, trimethylamine, diisopropylethylamine, N,N-dimethylaniline, N,N-diethylaniline, N-methylmorpholin and the like; a quaternary ammonium hydroxide such as tetrabutylammonium hydroxide and the like. From an industrial point of view, those employed preferably are alkaline metal carbonates, alkaline metal hydrogen carbonates, alkaline metal hydroxides, alkoxyalkaline metal salts and the like, with potassium carbonate, potassium hydrogen carbonate, sodium hydroxide, potassium hydroxide, sodium methoxide and potassium methoxide being more preferred. Any of these may be employed alone or in combination of two or more.

While the amount of a base employed is not limited particularly, it is generally 0.8 equivalent or more based on thioacetic acid employed, more preferably 1.0 equivalent or more.

This reaction is conducted preferably in an inert gas atmosphere, generally under a nitrogen or argon atmosphere.

After completion of the reaction, a target compound can be obtained from the reaction mixture by an extraction with an ordinary extraction solvent. It is also preferable that the extract is further washed with water. The resultant extract is concentrated to obtain the target compound. While the target substance thus obtained is almost pure, it may further be purified for example by a column chromatography.

Preferred embodiments of the invention are described below, but are not intended to restrict the invention.

Firstly with regard to Step a), a case employing an (S)-2-benzyl-3-methanesulfonyloxy-1-propanol as a substrate to produce an (R)-2-benzyl-3-methanesulfonyloxypropionic acid is discussed.

In one preferred embodiment, an (S)-2-benzyl-3-methanesulfonyloxy-1-propanol is dissolved in a solvent mixture of ethyl acetate and water and then 3.0 equivalents (equivalents based on the substrate, the same applies analogously to the followings) of potassium permanganate was added under an acetic acidic condition to effect the reaction.

In another preferred embodiment, an (S)-2-benzyl-3-methanesulfonyloxy-1-propanol is dissolved in a solvent mixture of acetone and water and then 3.0 equivalents of potassium permanganate was added under an sulfuric acidic condition to effect the reaction.

Secondly with regard to Step b), a case employing an (R)-2-benzyl-3-methanesulfonyloxypropionic acid as a substrate to produce an (S)-2-benzyl-3-acetylthiopropionic acid is discussed.

In one preferred embodiment, an (R)-2-benzyl-3-methanesulfonyloxypropionic acid is reacted with 1.5 equivalents of potassium thioacetate under a nitrogen atmosphere in a biphasic system of a solvent mixture of water and toluene, or water and ethyl acetate at 40°C to obtain a target material.

In another preferred embodiment, an (R)-2-benzyl-3-methanesulfonyloxypropionic acid is reacted with 1.5 equivalents of potassium thioacetate under a nitrogen atmosphere in water as a solvent at 40°C to obtain a target material.

In a still another preferred embodiment, an (R)-2-benzyl-3-methanesulfonyloxypropionic acid is reacted with 1.5 equivalents of potassium thioacetate under a nitrogen atmosphere in a 50/50 (v/v) solvent mixture of water and methanol at 40°C to obtain a target material.

### BEST MODE FOR CARRYING OUT THE INVENTION

The invention is further described in the following EXAMPLES, which are not intended to restrict the invention.

The chemical purity analysis of Compounds (1), (2) and (3) was conducted using the following liquid chromatography.
Column: Manufactured by Nacalaitesque, COSMOSIL 5C18-ARII, 250 mm x 4.6 mm, I.D.5 µm
Eluent: 7.35 mM phosphate buffer (pH4)/acetonitrile=65/35(v/v)
Flow rate: 1.0 ml/min
Detection: UV210 nm
Column temperature: 40°C

The optical purity analysis of Compound (1) was conducted using the following liquid chromatography using the following optically active column.
Column: Manufactured by Daicell, CHIRALPAK AD, 250 mm x 4.6 mm, I.D.10 µm
Eluent: n-Hexane/2-propanol/trifluoroacetic acid=80/20/0.1(v/v/v) Flow rate: 1.0 ml/min
Detection: UV210 nm
Column temperature:40°C

The optical purity analysis of Compound (2) was conducted using the following liquid chromatography using the following optically active column.
Column: Manufactured by Daicell, CHIRALPAK AD, 250 mm x 4.6 mm, I.D.10 µm
Eluent: n-Hexane/2-propanol/trifluoroacetic acid=80/20/0.1(v/v/v) Flow rate: 0.5 ml/min
Detection: UV210 nm
Column temperature:40°C

The optical purity analysis of Compound (3) was conducted using the following liquid chromatography using the following optically active column.
Column: Manufactured by Daicell, CHIRALPAK AD, 250 mm x 4.6 mm, I.D.10 µm
Eluent: n-Hexane/2-propanol/trifluoroacetic acid=90/10/0.1(v/v/v) Flow rate: 0.5 ml/min
Detection: UV210 nm
Column temperature: 40°C

### EXAMPLE 1 (R)-2-Benzyl-3-methanesulfonyloxypropionic acid

2.44 g of (S)-2-benzyl-3-methanesulfonyloxy-1-propanol (10.0 mmol, optical purity 98.5%ee) was dissolved in a solvent mixture of 25 ml of water and 25 ml of ethyl acetate, which was combined with 12.0g of acetic acid (200.0 mmol, 20 equivalents), and 4.74g of potassium permanganate (30.0 mmol, 3.0 equivalents (equivalent to substrate, the same applies analogously to the followings)) was added to the mixture, which had been cooled at 5 C°, over 4 hours and reacted for additional 2 hours with stirring.

After completion of the reaction, 6.2 g of sodium hydrogen sulfite was added portionwise while keeping the mixture at pH 1 whilst adding 15% sulfuric acid at the same temperature, and then the mixture was warmed to 15 C° to obtain a clear biphasic mixture. The aqueous phase was separated, and the organic phase was washed twice with 20ml of water, and then the organic phase was concentrated under reduced pressure. The oily matter obtained contained 2.36 g of (R)-2-benzyl-3-methanesulfonyloxypropionic acid. Yield: 91.5%, purity: 97.5% or higher, optical purity: 98.5%ee. Impurities whose aromatic rings were halogenated were not found.

### REFERENCE EXAMPLE 1 (R)-2-Benzyl-3-methanesulfonyloxypropionic acid

2.29 g of (R)-2-benzyl-3-methanesulfonyloxypropionic acid obtained in example 1 was combined with 10ml of toluene, heated at 35 C°, and stirred to precipitate a crystal. The mixture was combined with 10ml of hexane, and then cooled to 25 C°, and a crystal precipitated was collected by filtration. The crystal was washed with a mixture of 5 ml of toluene and 10 ml of hexane, and dried under reduced pressure to obtain 2.20g of the target compound, (R)-2-benzyl-3-methanesulfonyloxypropionic acid. Yield: 96.0%, purity: 99.0% or higher, optical purity: 98.8%ee.

### EXAMPLE 2 (R)-2-Benzyl-3-methanesulfonyloxypropionic acid

2.44 g of (S)-2-benzyl-3-methanesulfonyloxy-1-propanol (10.0 mmol, optical purity 98.5%ee) was dissolved in a solvent mixture of 6 ml of water and 10 ml of ethyl acetate, which was combined with 6.0 g of acetic acid (100 mmol, 10 equivalents), and 4.74 g of potassium permanganate (30.0 mmol, 3.0 equivalents) was added to the mixture, which had been cooled at 5 C°, over 4 hours and reacted for additional 2 hours with stirring.

After completion of the reaction 4.2 g of sodium hydrogen sulfite was added portionwise while keeping the mixture at pH 1 whilst adding 15% of sulfuric acid at the same temperature, and then the mixture was warmed to 15 C° to obtain a clear biphasic mixture. The aqueous phase was separated, and the organic phase was washed twice with 5ml of water, and then the organic phase was concentrated under reduced pressure. The oily matter obtained contained 2.33 g of (R)-2-benzyl-3-methanesulfonyloxypropionic acid. Yield: 90.1%, purity: 97.5% or higher, optical purity: 98.5%ee. Impurities whose aromatic rings were halogenated were not found.

### EXAMPLE 3 (R)-2-Benzyl-3-methanesulfonyloxypropionic acid

Oily matter, containing 2.38 g of the target compound, (R)-2-benzyl-3-methanesulfonyloxypropionic acid, was obtained in the same manner as that of Example 1, excepting the coexistence of 0.64 g of tetra-n-butyl ammonium bromide (2.0 mmol) during the reaction. Yield: 92.0%, purity: 97.5% or higher, optical purity: 98.5%ee. Impurities whose aromatic rings were halogenated were not found.

### EXAMPLE 4 (R)-2-Benzyl-3-methanesulfonyloxypropionic acid

2.44 g of (S)-2-benzyl-3-methanesulfonyloxy-1-propanol (10.0 mmol, optical purity 98.5%ee) was dissolved in a solvent mixture of 25 ml of 15% sulfuric acid and 12.5 ml of acetone, and after cooling to 5 C°, 4.74 g of potassium permanganate (30.0 mmol, 3.0 equivalents) was added over 4 hours and reacted for additional 1 hour with stirring.

After completion of the reaction, 60 ml of ethyl acetate was added, and then 3.64 g of sodium hydrogen sulfite was added while keeping the same temperature to obtain a clear biphasic solution. At this time, the pH was 1. The aqueous phase was separated, and the organic phase was washed twice with 20ml of water, and then the organic phase was concentrated under reduced pressure. The oily matter obtained contained 2.34 g of the target compound, (R)-2-benzyl-3-methanesulfonyloxypropionic acid. Yield: 90.7%, purity: 97.5% or higher, optical purity: 98.5%ee. Impurities whose aromatic rings were halogenated were not found.

### EXAMPLE 5 (S)-2-Benzyl-3-acetylthiopropionic acid

90.0g of (R)-2-benzyl-3-methanesulfonyloxypropionic acid (0.348 mol, optical purity 98.5%ee) obtained similarly to reference example 1 was dissolved in 450 ml of water and 1350 ml of toluene, 59.7 g of potassium thioacetate (0.523 mol, 1.5 equivalents) was added under nitrogen atmosphere, and the mixture was reacted at 40 C° for 24 hours with stirring. After completion of the reaction, the mixture was cooled to 20 C° and acidified (pH 1) by an addition of 30.8 g of sulfuric acid. The aqueous phase was separated, and the organic phase was washed twice with 450 ml of water, and then the organic phase was concentrated under reduced pressure. The oily matter obtained contained 82.1 g of the target compound, (S)-2-benzyl-3-acetylthiopropionic acid. Yield: 99.0%, purity: 98.9%, deacetylated form: 0.1%, optical purity: 98.5%ee.

### EXAMPLE 6 (S)-2-Benzyl-3-acetylthiopropionic acid

10.0g of (R)-2-benzyl-3-methanesulfonyloxypropionic acid (38.7 mmol, optical purity 98.5%ee) obtained similarly to reference example 1 was dissolved in 50 ml of water and 50 ml of ethyl acetate, 6.6 g of potassium thioacetate (58.1 mmol, 1.5 equivalents) was added under nitrogen atmosphere, and the mixture was reacted at 40 C° for 24 hours with stirring. After completion of the reaction, the mixture was cooled to 20 C° and acidified (pH 1) by an addition of 3.5 g of sulfuric acid. The aqueous phase was separated, and the organic phase was washed twice with 25 ml of water, and then the organic phase was concentrated under reduced pressure. The oily matter obtained contained 9.1 g of the target compound, (S)-2-benzyl-3-acetylthiopropionic acid. Yield: 98.8%, purity: 98.4%, deacetylated form: 0.2%, optical purity: 98.5%ee.

### EXAMPLE 7 (S)-2-Benzyl-3-acetylthiopropionic acid

10.0g of (R)-2-benzyl-3-methanesulfonyloxypropionic acid (38.7 mmol, optical purity 98.5%ee) obtained similarly to reference example 1 was dissolved in 200 ml of water, 6.6 g of potassium thioacetate (58.1 mmol, 1.5 equivalents) was added under nitrogen atmosphere, and the mixture was reacted at 40 C° for 24 hours with stirring. After completion of the reaction, the mixture was cooled to 20 C°, combined with 200 ml of ethyl acetate, and acidified (pH 1) by an addition of 3.5 g of sulfuric acid. The aqueous phase was separated, and the organic phase was washed twice with 25 ml of water, and then the organic phase was concentrated under reduced pressure. The oily matter obtained contained 9.0 g of the target compound, (S)-2-benzyl-3-acetylthiopropionic acid. Yield: 97.5%, purity: 97.0%, deacetylated form: 0.6%, optical purity: 98.5%ee.

### EXAMPLE 8 (S)-2-Benzyl-3-acetylthiopropionic acid

10.0g of (R)-2-benzyl-3-methanesulfonyloxypropionic acid (38.7 mmol, optical purity 98.5%ee) obtained similarly to reference example 1 was dissolved in 100 ml of water and 100 ml of methanol, 6.6 g of potassium thioacetate (58.1 mmol, 1.5 equivalents) was added under nitrogen atmosphere, and the mixture was reacted at 40 C° for 24 hours with stirring. After completion of the reaction, the mixture was cooled to 20 C°, combined with 200ml of ethyl acetate, and acidified (pH 1) by an addition of 3.5 g of sulfuric acid. The aqueous phase was separated, and the organic phase was washed twice with 25 ml of water, and then the organic phase was concentrated under reduced pressure. The oily matter obtained contained 8.9 g of the target compound, (S)-2-benzyl-3-acetylthiopropionic acid. Yield: 96.8%, purity: 96.3%, deacetylated form: 1.7%, optical purity: 98.5%ee.

### EXAMPLE 9 (S)-2-Benzyl-3-acetylthiopropionic acid

10.0g of (R)-2-benzyl-3-methanesulfonyloxypropionic acid (38.7 mmol, optical purity 98.5%ee) obtained similarly to reference example 1 was dissolved in 40 ml of water and 160 ml of methanol, 6.6 g of potassium thioacetate (58.1 mmol, 1.5 equivalents) was added under nitrogen atmosphere, and the mixture was reacted at 40 C° for 24 hours with stirring. After completion of the reaction, the methanol was distilled off under reduced pressure, and the mixture was combined with 100 ml of ethyl acetate at 20 C° and acidified (pH 1) by an addition of 3.5 g of sulfuric acid. The aqueous phase was separated, and the organic phase was washed twice with 25 ml of water, and then the organic phase was concentrated under reduced pressure. The oily matter obtained contained 8.8 g of the target compound, (S)-2-benzyl-3-acetylthiopropionic acid. Yield: 95.8%, purity: 95.7%, deacetylated form: 2.6%, optical purity: 98.5%ee.

### COMPARATIVES 1-10 (S)-2-Benzyl-3-acetylthiopropionic acid

1.0g of (R)-2-benzyl-3-methanesulfonyloxypropionic acid (3.87 mmol) and 0.66 g of potassium thioacetate (5.81 mmol, 1.5 equivalents) were dissolved in 10 ml of each solvent described in the following table, and the mixture was reacted at 40 C° for 7 hours under nitrogen atmosphere, and then it was shown that the starting compounds were disappeared. After completion of the reaction the mixture was cooled to 20 C°, combined with 50 ml of ethyl acetate and 10 ml of water, and acidified (pH 1) by an addition of sulfuric acid. After separation of the aqueous phase, the organic phase was washed with 10 ml of pure water and the organic phase was concentrated under reduced pressure to obtain oily matter, which was analyzed by a liquid chromatography to find the ratio of the target compound, (S)-2-benzyl-3-acetylthiopropionic acid to the total amount of impurities (HPCL; % area). The results were shown in Table 1.

**Table 1**

| Comparative | Solvent | Ratio of target compound: total impurities |
|---|---|---|
| 1 | Methanol | 95:05 |
| 2 | Ethanol | 90:10 |
| 3 | 2-Propanol | 79:21 |
| 4 | Dimethylformamide | 69:31 |
| 5 | Toluene | 59:41 |
| 6 | Ethyl acetate | 57:43 |
| 7 | Acetonitrile | 55:45 |
| 8 | Acetone | 52:48 |
| 9 | 1,4-Dioxane | 49:51 |
| 10 | Tetrahydrofuran | 45:55 |

### INDUSTRIAL APPLICABILITY

The invention has the aspects described above, and can provides a method for producing a 2-aralkyl-3-acetylthiopropionic acid and a 2-aralkylpropionic acid having a sulfonyloxy group or a halogen atom in the 3-position, especially an optically active 2-aralkyl-3-acetylthiopropionic acid and an optically active 2-aralkylpropionic acid having a sulfonyloxy group or a halogen atom in the 3-position which are very useful as production intermediates employed in the fields of pharmaceuticals and others in an industrially advantageous and convenient manner at a high purity.

## Claims

1. A method for producing a 2-aralkylpropionic acid represented by Formula (2): 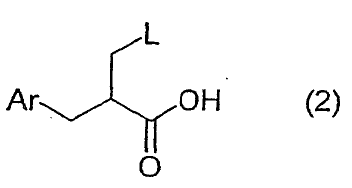 wherein Ar is an optionally substituted aryl group having 6 to 18 carbon atoms, and L is a sulfonyloxy group or a halogen atom, comprising oxidizing a 2-aralkyl-1-propanol represented by Formula (1): 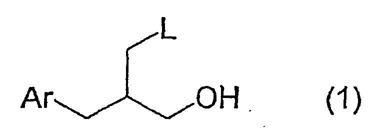 wherein Ar and L are as defined above, using a permanganate under an acidic condition.

2. The method according to Claim 1 wherein Ar is an optionally substituted phenyl group or an optionally substituted naphthyl group.

3. The method according to Claim 1 wherein Ar is an optionally substituted phenyl group.

4. The method according to any one of Claims 1 to 3 wherein L is an optionally substituted straight, branched or cyclic alkylsulfonyloxy group having 1 to 6 carbon atoms or an optionally substituted arylsulfonyloxy group having 6 to 18 carbon atoms.

5. The method according to any one of Claims 1 to 3 wherein L is a methanesulfonyloxy group or a toluenesulfonyloxy group.

6. The method according to Claim 1 wherein Ar is a phenyl group and L is a methanesulfonyloxy group.

7. The method according to any one of Claims 1 to 3 wherein L is a halogen atom.

8. The method according to any one of Claims 1 to 7 wherein the permanganate is an alkaline metal salt of permanganic acid.

9. The method according to Claim 8 wherein the alkaline metal salt of permanganic acid is potassium permanganate.

10. The method according to any one of Claims 1 to 9 wherein the acidic condition is formed in acidic aqueous solution consisting of water and acetic acid or water and sulfuric acid.

11. The method according to any one of Claims 1 to 10 wherein a solvent mixture of the acidic aqueous solution and an organic solvent is employed.

12. The method according to Claim 11 wherein the organic solvent is an organic solvent having no compatibility with water and the reaction is conducted in a biphasic system with the acidic aqueous solution.

13. The method according to Claim 12 wherein the organic solvent having no compatibility with water is an acetic alkyl ester having 1 to 6 carbon atoms.

14. The method according to Claim 13 wherein the acetic alkyl ester having 1 to 6 carbon atoms.

15. The method according to Claim 11 wherein the organic solvent is an organic solvent having a compatibility with water.

16. The method according to Claim 15 wherein the organic solvent having a compatibility with water is acetone, tetrahydrofuran or tert-butanol.

17. The method according to Claim 16 wherein the acidic aqueous solution consists of sulfuric acid and water and the reaction is conducted in a solvent mixture system with acetone.

18. The method according to any one of Claims 1 to 17 wherein a treatment with a reducing agent is conducted under an acidic condition after the reaction.

19. The method according to Claim 18 wherein the reducing agent is a hydrogen sulfite, sulfite, pyrosulfite or an aqueous solution thereof.

20. A method for producing a 2-aralkyl-3-acetylthiopropionic acid represented by Formula (3): 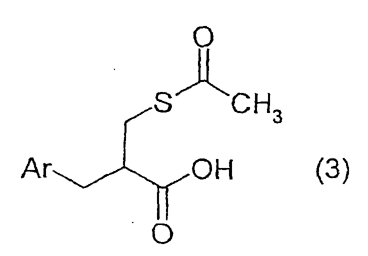 wherein Ar is an optionally substituted aryl group having 6 to 18 carbon atoms comprising reacting a 2-aralkylpropionic acid represented by Formula (2): 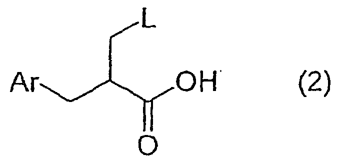 wherein Ar is as defined above and L is a sulfonyloxy group or a halogen atom with a thioacetate in the presence of water.

21. The method according to Claim 20 wherein Ar is an optionally substituted phenyl group or an optionally substituted naphthyl group.

22. The method according to Claim 20 wherein Ar is an optionally substituted phenyl group.

23. The method according to any one of Claims 20 to 22 wherein L is an optionally substituted straight, branched or cyclic alkylsulfonyloxy group having 1 to 6 carbon atoms or an optionally substituted arylsulfonyloxy group having 6 to 18 carbon atoms.

24. The method according to any one of Claims 20 to 22 wherein L is a methanesulfonyloxy group or a toluenesulfonyloxy group.

25. The method according to Claim 20 wherein Ar is a phenyl group and L is a methanesulfonyloxy group.

26. The method according to any one of Claims 20 to 22 wherein L is a halogen atom.

27. The method according to any one of Claims 20 to 26 wherein the reaction solvent is water.

28. The method according to any one of Claims 20 to 26 wherein the reaction solvent is a solvent mixture of water and an organic solvent.

29. The method according to Claim 28 wherein the organic solvent is an organic solvent having no compatibility with water and the reaction is conducted in a biphasic system with water.

30. The method according to Claim 29 wherein the organic solvent having no compatibility with water is an aromatic hydrocarbon or an acetic alkyl ester having 1 to 6 carbon atoms.

31. The method according to Claim 30 wherein the organic solvent having no compatibility with water is toluene or ethyl acetate.

32. The method according to Claim 28 wherein the organic solvent is an organic solvent having a compatibility with water.

33. The method according to Claim 32 wherein the organic solvent having a compatibility with water is alcohol having 1 to 3 carbon atoms.

34. The method according to Claim 33 wherein the organic solvent having a compatibility with water is methanol.

35. The method according to any one of Claims 20 to 34 wherein the thioacetate is an alkaline metal salt of thioacetic acid.

36. The method according to Claim 35 wherein the alkaline metal salt of thioacetic acid is potassium thioacetate.

37. The method according to any one of Claims 20 to 36 wherein the thioacetate is formed in situ using thioacetic acid and a base.

38. The method according to any one of Claims 20 to 37 wherein the reaction is conducted under an inert gas atmosphere.

39. The method according to any one of Claims 20 to 38 wherein a 2-aralkylpropionic acid represented by Formula (2): 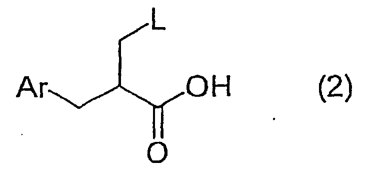 wherein Ar and L are as defined above, is obtained by oxidizing a 2-aralkyl-1-propanol represented by Formula (1): 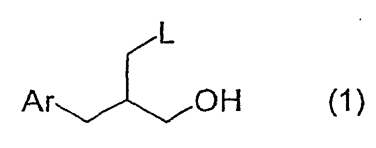 wherein Ar and L are as defined above, using a permanganate under an acidic condition.

40. The method according to Claim 39 wherein the permanganate is an alkaline metal salt of permanganic acid.

41. The method according to Claim 40 wherein the alkaline metal salt of permanganic acid is potassium permanganate.

42. The method according to any one of Claims 39 to 41 wherein the acidic condition is formed in an acidic aqueous solution consisting of water and acetic acid or water and sulfuric acid.

43. The method according to any one of Claims 39 to 42 wherein a solvent mixture of the acidic aqueous solution and an organic solvent is employed.

44. The method according to Claim 43 wherein the organic solvent is an organic solvent having no compatibility with water and the reaction is conducted in a biphasic system with the acidic aqueous solution.

45. The method according to Claim 44 wherein the organic solvent having no compatibility with water is an acetic alkyl ester having 1 to 6 carbon atoms.

46. The method according to Claim 45 wherein the acidic aqueous solution consists of water and acetic acid and the reaction is conducted in a biphasic system of ethyl acetate and the solvent mixture.

47. The method according to Claim 43 wherein the organic solvent is an organic solvent having a compatibility with water.

48. The method according to Claim 47 wherein the organic solvent having a compatibility with water is acetone, tetrahydrofuran or tert-butanol.

49. The method according to Claim 48 wherein the acidic aqueous solution consists of sulfuric acid and water and the reaction is conducted in a solvent mixture system with acetone.

50. The method according to any one of Claims 39 to 49 wherein the reaction from a compound represented by Formula (1) to a compound represented by Formula (2) is followed by a treatment with a reducing agent under an acidic condition.

51. The method according to Claim 50 wherein the reducing agent is a hydrogen sulfite, sulfite, pyrosulfite or an aqueous solution thereof.
